(19) Europäisches Patentamt

European Patent Office

Office européen des brevets

(11) **EP 1 060 748 A1**

(12) **EUROPEAN PATENT APPLICATION**
published in accordance with Art. 158(3) EPC

(43) Date of publication:
20.12.2000 Bulletin 2000/51

(51) Int. Cl.$^7$: **A61K 38/57**

(21) Application number: 99906472.8

(86) International application number:
PCT/JP99/00853

(22) Date of filing: 25.02.1999

(87) International publication number:
WO 99/43347 (02.09.1999 Gazette 1999/35)

(84) Designated Contracting States:
AT BE CH CY DE DK ES FI FR GB GR IE IT LI LU MC NL PT SE

(30) Priority: 25.02.1998 JP 5906398
22.05.1998 JP 14150098
30.06.1998 JP 18480198
09.10.1998 JP 28806398

(71) Applicant:
Wakamoto Pharmaceutical Co., Ltd.
Chuo-ku, Tokyo 103-8330 (JP)

(72) Inventors:
• TAKAHASHI, Ryoki
Hadano-shi Kanagawa 257-0003 (JP)
• NOGUCHI, Takayasu
Ashigarakami-gun Kanagawa 258-0018 (JP)
• AKIBA, Kiyoshi
Ashigarakami-gun Kanagawa 258-0111 (JP)
• OMURA, Takeo
Ashigarakami-gun Kanagawa 258-0019 (JP)

(74) Representative:
Forstmeyer, Dietmar, Dr. rer. nat., Dipl.-Chem. et al
Patentanwaltskanzlei Dr. Dietmar Forstmeyer,
Bereiteranger 15
81541 München (DE)

(54) **REMEDIES FOR CORNEAL EPITHELIUM DISTURBANCE**

(57)    The present invention has for its object to provide a therapeutic composition which acts on various phases of corneal epithelial wound healing with safety and without risks for side effects and can therefore enable an effective prevention or treatment of disorders of the corneal epithelium.

This invention is related to a pharmaceutical composition corneal epithelial disorders which is indicated in corneal epithelial disorders for the prophylaxis and/or therapy of corneal epithelial disorders,
comprising urinastatin as an active ingredient.

EP 1 060 748 A1

**Description**

TECHNICAL FIELD

The present invention relates to a therapeutic composition comprising urinastatin as an active ingredient, and more particularly to therapeutic compositions for the prophylaxis and therapy of corneal epithelial disorders, UV-induced keratoconjunctival epithelial disorders, keratectomy postoperative corneal epithelial complications, drug-induced keratoconjunctival epithelial disorders, and dry eye.

BACKGROUND ART

The cornea is a transparent apparatus situated over the anterior chamber of the lens at the anterior pole of the eyeglobe and having an orderly laminar structure composed of the epithelium, Bowman's membrane, stroma, Descemet's membrane and endothelium. The conjunctiva, which is continuous with the cornea, covers the surface of the globe and the inner surface of the eyelid. Morphologically the conjunctiva can be classified into the palpebral conjunctiva, fornical conjunctiva and bulbar conjunctiva but histologically it consists of an epithelial layer and a stromal layer. In this specification, the cornea and the conjunctiva are sometimes referred to collectively as the "keratoconjunctiva".

It is thought that disorders of the keratoconjunctiva occur as the result of lesion of its epithelium. Etiologically, keratoconjunctival disorders may be ascribed to certain endogenous diseases such as herpetic keratitis, bacterial corneal ulcer, neuroparalytic keratitis, diabetic keratopathy, Sjögren's syndrome, Stevens-Johnson syndrome, keratoconjunctivitis sicca (dry eye), etc. or exogenous diseases such as postoperative, drug-induced, traumatic, and contact lens wearer's diseases, or may be caused by physical or chemical invasions.

Among such keratoconjunctival disorders, corneal epithelial injury is a matter of concern because, depending on etiology, the injury may progress to refractory diseases of the cornea in which regeneration of the epithelium cannot be expected and the outcome of which is loss of vision or blindness.

For the recovery of vision in such cases, it is necessary that the normal regeneration and restoration of integrity of the corneal epithelium should take place rapidly.

The mechanism of healing of corneal epithelial wounds is comprised of the first to third phases. The first phase consists of epithelial cell adhesion, extension and migration, the second phase is characterized by mitotic proliferation of the epithelial cells, and the third phase by differentiation of the epithelial cells, with the orderly laminar structure of the epithelium being restored through this third phase.

Among the known modulating/controlling factors in the healing of corneal epithelial wounds are fibronectin, hyaluronic acid, IL-6, epidermal growth factor (EGF), etc. which promote the above-mentioned processes involved in the first phase. Moreover, Substance P joins forces with EGF or insulin-like growth factor (IGF-1) to promote the extension and migration of epithelial cells.

As the substances which promote the proliferation of epithelial cells in the second phase, EGF, FGF (fibroblast growth factor), keratinocyte growth factor (KGF) and hepatocyte growth factor (HGF), etc. are known [Gospodarowicz, D. et al.: Exp. Eye Res., 25, 631-649 (1977); Sotozono, C. et al.: Exp. Eye Res., 59, 385-392 (1994); Wilson, S. E. et al.: Ophthalmol. Vis. Sci., 34, 2544-2561 (1993)].

It is known that the differentiation of epithelial cells in the third phase is promoted by vitamin A.

Among such substances, those in use clinically as positive therapeutic drugs for corneal epithelial wounds are topical preparations of fibronectin and hyaluronic acid. It has been confirmed that both fibronectin and hyaluronic acid promote the adhesion and extension of corneal epithelial cells and that fibronectin, in particular, exhibits marked efficacy at a very low concentration.

However, the action of fibronectin and hyaluronic acid is primarily expressed in the processes involved in the first phase of corneal epithelial wound healing, and their contribution to mitotic proliferation of epithelial cells in the second phase is almost negligible. Moreover, growth factors such as EGF, FGF, KGF and HGF have the risk of inducing neovascularization which is considered to be an insurmountable deterrent to their application as medicines [Youji Mitsui et al.: Mechanism of Neovascularization; Ophthalmology New Insight, Vol. 3, Intraocular Angiogenic Diseases, 8-20, Medical View Co. (1994)].

In fact, there are reports on the neovascularization induced by EGF and FGF [Schweigerer, L.: Z. Kardiol., 78, 12-15 (1989); Taniguchi, E. et al.: Nippon Ganka Gakkai Zasshi, 95, 52-58 (1991); Nezu, E. et al.: Jpn. J. Ophthalmol., 36, 401-406 (1992)]. Furthermore, the considerable difficulties encountered in the production of those growth factors are also substantial impediments to their development as pharmaceuticals.

Thus, for use as the active ingredient of a therapeutic drug for corneal epithelial disorders, all the compounds so far known are unsatisfactory and a real demand exists for better compounds.

Meanwhile, it has been suspected for years that proteases including collagenase are associated with the pathogenesis of corneal ulcer in a significant measure [Motokazu Itoi: Journal of Clinical Ophthalmology, 24, 879-882 (1970)]. In fact, it is reported that a collagenase inhibitor such as cysteine suppresses experimental corneal ulcers and its clinical application has also been reported [Brown, S. I. et al.: Arch. Ophthalmol., 82, 95-97 (1969); Brown, S. I. et al.: Arch. Ophthalmol., 83, 352-353 (1970); Junzo Hirano et al.: Journal of Clinical Ophthalmology, 29, 49-54 (1975); Hajime Nunomura et al.: Journal of Clinical Ophthalmology, 71, 88-90 (1975)]. However, the therapeutic rewards they provide are not as remarkable as desired.

Meanwhile, the migration of epithelial cells is so important to the first phase of corneal epithelial wound healing and, as generally believed, the localization of proteases at the apical ends of extending cells is essential to said cell migration. Therefore, the relationship of the extension and migration of corneal epithelial cells to the proteases has heretofore been studied using protease inhibitors.

Morimoto et al. did investigations using various protease inhibitors and found that leupeptin (thiol protease inhibitor), aprotinin (serine protease inhibitor) and ovo$\alpha_2$-macroglobulin (general protease inhibitor) invariably suppress the extension of corneal epithelial cells [Keisuke Morimoto et al..: Collagen Study Group Research Abstract, 35, 170-173 (1987)].

The above finding was endorsed by the work of Zieske et al. who used, as protease inhibitors, pepstatin (acid protease inhibitor), 1,10-phenanthroline (metalloprotease inhibitor), aprotinin (serine protease inhibitor) and phenylmethylsulfonyl fluoride [Zieske, J. D. & Bukusoglu, G.: Investigative Ophthalmology & Visual Science, 32, 2073-2078 (1991)]. In any event, it has been suggested that protease inhibitors have an aspect of exerting a negative effect on corneal epithelial wound healing.

However, it is known that certain protease inhibitors, for example the proteinaceous protease inhibitors aprotinin, $\alpha_1$-antitrypsin, etc. may function as growth factors for human fibroblast cells and human endothelial cells [Ogawa, M. et al.: Res Commun. Chem. Pathol. Pharmacol. 50, 155-158 (1985); Scott, G. K. et al.: Biol. Chem. Hoppe-Seyler, 369, 131-135 (1988); Mckeehan, W. L. et al.: J. Biol. Chem., 261, 5378-5383 (1986)].

Furthermore, it is known that the plasminogen activator/plasmin system is essential to the collagenase production involved in the development of said corneal ulcer [Berman, M. et al.: Invest. Ophthalmol. Vis. Sci., 19, 1204-1221 (1980)]. Therefore, it has been postulated that plasmin inhibitors have healing effects on impaired corneal epithelium.

It was known for years that aprotinin is a potent plasmin inhibitor [Feeney, R. E. et al.: J. Biol. Chem., 25, 1957-1960 (1969)]. Actually, aprotinin was applied to the treatment of corneal epithelial wounds in man with more or less success [Japanese Kokoku Publication Hei-7-72139; USP 4,849,406; EP 0 223 254 B1; Salonen, E. M. et al.: Acta Ophthalmologia, 65, 3-12 (1987)].

However, as pointed out above, aprotinin rather acts in an inhibitory way on the migration of corneal epithelial cells which is of paramount importance to the treatment of corneal epithelial injuries [Zieske, J. D. & Bukusoglu, G.: Investigative Ophthalmology & Visual Science, 32, 2073-2078 (1991)] and, in addition, this substance has risks for inducing neovascularization, so that it has not come into general use as a therapeutic drug for impaired corneal epithelium.

It has recently been made clear that maintenance of plasminogen activator activity is important to the healing of corneal wounds [Morimoto, K. et al.: Thrombosis and Haemostasis 69, 387-391 (1993)]. Therefore, demands exist for a therapeutic drug for corneal epithelial disorders which does not act in an inhibitory way on plasmin and plasminogen activator or, if it does, is only moderate in inhibitory activity or has substantially no inhibitory action. The fact that aprotinin is a potent inhibitor of plasmin is also one of the reasons why this substance could not be developed as a pharmaceutical entity.

There are reports on the use of ovomacroglobulin, a proteinaceous protease inhibitor which inhibits other proteases inclusive of collagenase strongly and with broad specificity, in the treatment of refractory corneal diseases with limited success [Ryuji Kamata et al.: Journal of Clinical Ophthalmology, 45, 233-237 (1991); Ryuji Kamata et al.: Nippon-no-Ganka, 64, 955-960 (1993)] but for the reason, among others, that, being a foreign protein derived from hen's egg white, the ovomacroglobulin used had the risk for antigenicity and allergic reactions, this substance could not be applied clinically with success as a therapeutic drug for corneal epithelial disorders.

These reports suggested the possibility of proteinaceous protease inhibitors promoting proliferation of human corneal epithelial cells and thereby exhibiting efficacy in wound healing but because they inhibit plasmin and plasminogen activator and, in addition, have fatal side effects such as neovascularization and antigenicity, there has been a standing need for the discovery of a more satisfactory compound which might be developed into a pharmaceutical product for administration to humans.

Meanwhile, among keratoconjunctival diseases, the disorder attributable to ultraviolet radiation is a focus of attention today. On occasions where the eye is exposed to solar radiation, such as sea bathing, ultraviolet rays impinge on the corneal epithelium and, through various factors, induce disorders of the keratoconjunctival epithelia.

As UV-induced corneal epithelial disorders, the following are known.

Photokeratitis accompanied by necrosis and erosion [Bergmanson J. P.: Corneal damage in photokeratitis - Why is it so painful?; Optom. Vis. Sci., 67, 407-413, 1990]; "snow blindness" characterized by pain, photophobia and blepharospasm; UV-induced cataract in which a plurality of etiologic factors are involved [Hightower K. R.: A review of the evidence that ultraviolet irradiation is a risk factor in cataractogenesis; Doc. Ophthalmol., 88, 205-220, 1994]; immunosuppression associated with damage to Langerhans cells [Kelley J. G. et al: Langerhans cell alterations in the guinea pig cornea; Invest. Ophthalmol. Vis. Sci., 26, 1293-1296, 1985; Ray-Kell L. et al.: Reduction in the incidence of rejection of heterotopic murine corneal transplants by pretreatment with ultraviolet radiation; Transplantation, 42, 403-406, 1986];

induction of inflammatory cytokines [Kennedy M. et al: Ultraviolet irradiation induces the production of multiple cytokines by human corneal cells; Invest. Ophthalmol. Vis. Sci., 38, 2483-2491, 1997]; DNA damage [Reddy, V. N. et al: The effect of aqueous humor ascorbate on ultraviolet-B-induced DNA damage in lens epithelium; Invest. Ophthalmol. Vis. Sci., 39, 344-350, 1998]; and neuropathy [Trabucchi, G. et al.: Corneal nerve damage and regeneration after excimer laser photokeratectomy in rabbit eyes; Invest. Ophthalmol. Vis. Sci., 35, 229-235, 1994], among others.

Nearly all of these diseases are accompanied by damage to the keratoconjunctival epithelial cells, and the injury inflicted by free radicals such as superoxide, hydroxy radical, hydroperoxide, etc. as produced by the influence of UV radiation is considered to be a major cause.

When attention is paid to the mechanism for homeostasis of a living body, it may be postulated that the lacrimal fluid inherently contains sufficient amounts of lactoferrin (about 2 mg/ml tear) and glutathione to quench or inhibit such free radicals.

Therefore, although the various UV-induced disorders of the keratoconjunctival epithelia are triggered by the free radicals formed by UV, it is logical to consider that a more crucial cause of disease exists in the mechanism for homeostasis of the living body.

From the above point of view, a drug having both a free radical quenching or inhibitory action and a function to encourage the defense mechanism of the body should contribute to the prevention and treatment of the UV-induced damage to the keratoconjunctival epithelia.

As physiological topical ophthalmic drugs having defensive potentials against UV-induced disorders, glutathione, vitamin C, vitamin E, etc. are known. Moreover, it is known that lactoferrin and glutathione, both of which are physiological ingredients of lacrimal fluid, quench free-radical, and protect the keratoconjunctival epithelia against injury [Megaw J. M.: Glutathione and ocular photobiology; Curr. Eye Res., 3, 83-87, 1984].

Furthermore, application of UV-blocking contact lenses has also been attempted [Bergmanson, J. P.: The significance of ultraviolet radiation for eye diseases, A review with comment on the efficacy of UV-blocking contact lenses; Ophthalmic. Physiol. Opt., 15, 83-91, 1995].

However, the predominant function of those known drugs, ingredients and devices is to protect the eye by quenching the free radicals generated by ultraviolet radiation and none are satisfactory enough from the standpoint of maintaining the homeostasis of the eye.

Meanwhile, as a method of correcting for myopia, refractive keratectomy has received much attention in recent years. It is a time-honored practice to correct for myopia by wearing spectacles or contact lenses but the use of spectacles is accompanied by the beauty problem and troublesomeness in daily living, while use of the contact lens tends to cause accidental corneal injury. Therefore, a radical method of correction for myopia has been needed.

In contrast, refractive keratectomy enables correction for myopia by a single operation and dispenses with the trouble of wearing spectacles or contact lenses, so that an interest is mounting on this operation as an epochal procedure.

However, depending on the skill and expertise, refractive keratectomy may entail corneal epithelial disorders and, therefore, establishment of a treatment protocol for the prophylaxis and therapy of such disorders has come to be a research objective as an indispensable prerequisite to the establishment of refractive keratectomy as a routine technique.

Refractive keratectomy is an operation performed to correct for myopia which comprises incising the superficial layer of the cornea to a predetermined pattern and size according to the correction required and is generally known as RK for short.

In the RK procedure, a technique using a diamond knife has been used for incising the cornea. According to this technique, the patient is directed to keep gazing at a fixation lamp mounted on a dissecting microscope and the operator incises the cornea free-hand.

In such cases, steroids and antibiotics are instilled in the eye postoperatively for prevention of infection and improved prognosis.

Another technique for refractive keratectomy is known as photo refractive keratectomy (PRK) in which a corneal incision is made with an excimer laser beam.

PRK does not depend on the operator's manipulative skill for the accuracy of an operation and, therefore, is attracting much attention as a preferred technique for refractive keratectomy in which the postoperative course may vary markedly with a subtle difference in the pattern and length of an incision. Moreover, the necessary equipment is also undergoing steady sophistication. It is expected that the current procedures for refractive keratectomy will ultimately converge into PRK.

PRK is an operation in which an excimer laser beam is focused on the corneal epithelium to make an incision in the irradiated position. Since this technique enables not only correction for myopia but also correction for hyperopia and astigmatism, it is considered to be a very instrumental technique for visual correction. This kind of procedure has recently come to be called refractive surgery.

While excimer layer PRK has been confirmed to show an excellent corrective efficacy, a variety of complications have also been reported. For example, corneal epithelial defects are caused in the various laminae and these are more or less unavoidable in excimer layer surgery and sometimes accompanied by severe pain.

Furthermore, subepithelial haze is invariably observed during a certain postoperative period. Although this haze disappears within 12 months of surgery, it is considered to be a matter of concern in many cases because an impaired vision cannot be avoided during the intervening period. Moreover, a significant decrease in the cell population is observed in the corneal endothelium.

Therefore, it is indispensable to overcome these postoperative complications in order that PRK using an excimer laser may enjoy a broader usage.

Since the excimer laser uses an ultraviolet emission with a wavelength of 193 nm, it was foreseeable that the problems mentioned above could resolve themselves if a substance be discovered that would increase the tolerance of corneal epithelial cells to the ultraviolet radiation at 193 nm.

Meanwhile, as the drugs for the prophylaxis, diagnosis and/or therapy of diseases of the eye, which are collectively referred to as ophthalmic drugs, a variety of oral medicaments and drugs for topical instillation have been developed and applied clinically.

The diseases of the eye in which such drugs are indicated include glaucoma, cataract, keratopathy, scleral diseases, retinopathy, uveal diseases, vitreous diseases, optic nerve diseases, conjunctival diseases, lacrimal apparatus diseases, orbital disorders and asthenopia or eyestrain, among others. And these drugs have been used in many kinds of surgeries, preoperatively, intraoperatively or postoperatively.

As the drugs for use in the prophylaxis, diagnosis and/or therapy of such diseases, there may be mentioned antiglaucoma drugs, anticataract drugs, antimicrobials (antibiotics, synthetic antibacterial agents), adrenocortical hormones, mydriatics, miotics, sulfa drugs, local anesthetics, vasoconstrictors, vasodilators, astringents, enzyme preparations, antiallergic agents, nonsteroidal antiinflammatory agents, antifungal agents, antiviral agents, and corneal protectant/healing promoters, among others.

Some of these ophthalmic drugs, despite their effectiveness in diseases of the eye, induce serious keratoconjunctival epithelial disorders as side effects. Drugs having such side effects cannot be used as valid ophthalmic drugs. Therefore, there has been a demand for a substance which should be capable of masking such side effects on concurrent administration with said drugs and can be used as a useful ophthalmic drug.

Meanwhile, one of keratoconjunctival diseases that has been attracting particular attention in recent years is dry eye. The incidence of dry eye is tending to increase in parallel with the increasing population wearing the contact lens, the increasing number of hours of living in an artificially air-conditioned environment, and the increasing number of occasions that one fixes a gaze on the television or computer VDT screen and has come to be a matter of serious concern.

Dry eye means a decrease in tear volume or a qualitative abnormality of tears, without regard to the presence or absence of a keratoconjunctival impairment [Yamada et al.: Folia Ophthalmologica Japonica, 43, 1289-1293 (1992)]. According to this definition, lacrimal deficiency, hypolacrimia, xerophthalmia, Sjögren's syndrome, Stevens-Johnson syndrome, pemphigus, diseases of lid margin, lid closure insufficiency, sensory nerve palsy, etc. are subsumed in the category of dry eye.

A relationship of a dry eye to the keratoconjunctival epithelial mucin has been pointed out and many investigations have been undertaken. Mucin 1 is known as the substance elaborated by both the corneal epithelium and the conjunctival epithelium excepting goblet cells [Journal of the Eye, Vol. 14, No. 11 (1997)]. As a therapeutic modality for a dry eye, instillation of artificial tears containing a viscoelastic substance, such as methylcellulose, chondroitin sulfate, hyaluronic acid or the like, as a substitute for mucin has been performed. However, because these substances differ from mucin in physical and physiological properties, the therapeutic reward they provide is limited.

International Patent WO97/39769 describes an attempt to use a system containing albumin as an active ingredient in dry eyes. This approach is intended to augment mucin secretions from the superficial epithelium and thereby stabilize the tear film on the eye surface.

Japanese Kokai Publication Hei-9-136832 discloses a technology of providing a prophylactic and/or therapeutic drug for dry eyes through formulation of sulfodehydroabietic acid. This agent potentiates the mucopolysaccharide-producing function of the conjunctival goblet cells which are mucin-producing cells and inhibits keratose change of the keratoconjunctiva due to the detraction from the function of said goblet cells.

Japanese Kokai Publication Hei-9-301866 discloses a dry eye remedy containing a carbostyril derivative. This drug increases the number of goblet cells to thereby increase mucin production as well as the volume of mucous fluid of the eye.

Japanese Kokai Publication Hei-10-218792 discloses a dry eye remedy containing an angiotensin-converting enzyme inhibitor. This drug exploits the fact that the angiotensin-converting enzyme inhibitor functions as a neurergic drug acting directly on the lacrimal gland function to promote secretion of tears.

These technologies are either not fully therapeutically effective or entail side effects and, as such, have the disadvantage that they do not contribute to a radical therapy.

SUMMARY OF THE INVENTION

The present invention, developed in view of the above state of the art, has for its object to provide a drug which acts on various phases of corneal epithelial wound healing with safety and without risks for side effects and can therefore enable an effective prevention or treatment of disorders of the corneal epithelium.

It is a further object of the present invention to provide a therapeutic composition for UV-induced keratoconjunctival epithelial disorders, which is effective in the prophylaxis and therapy of keratoconjunctival epithelium damages caused by UV radiation.

It is another object of the present invention to provide a drug for which is effective in preventing postoperative complications in excimer laser PRK cases.

It is a still another object of the present invention to provide a therapeutic composition for keratoconjunctival epithelial disorders, which eliminates the side effects of other ophthalmic drugs on the keratoconjunctival epithelia and thereby enables safe use of said ophthalmic drugs in said indications.

It is a further object of the present invention to provide a drug, which is capable of getting rid of etiologic factors in dry eye and thereby enables the prophylaxis and therapy of dry eyes.

The first aspect of the present invention is concerned with a therapeutic composition for corneal epithelial disorders which is indicated for the prophylaxis and/or therapy of corneal epithelial disorders,

comprising urinastatin as an active ingredient.

The second aspect of the present invention is concerned with a therapeutic composition for UV-induced keratoconjunctival epithelial disorders which is indicated for the prophylaxis and/or therapy of UV-induced keratoconjunctival epithelial disorders,

comprising urinastatin as an active ingredient.

The third aspect of the present invention is concerned with a therapeutic composition for keratectomy postoperative corneal epithelial complications which is indicated for the prophylaxis and/or therapy of keratectomy postoperative corneal epithelial complications,

comprising urinastatin as an active ingredient.

The fourth aspect of the present invention is concerned with a therapeutic composition for drug-induced keratoconjunctival epithelial disorders which is indicated for the therapy of keratoconjunctival epithelial disorders caused by administration of ophthalmic drugs,

comprising urinastatin as an active ingredient.

The fifth aspect of the present invention is concerned with a therapeutic composition for dry eyes which is indicated for the prophylaxis and/or therapy of dry eyes,

comprising urinastatin as an active ingredient.

BRIEF DESCRIPTION OF THE DRAWING

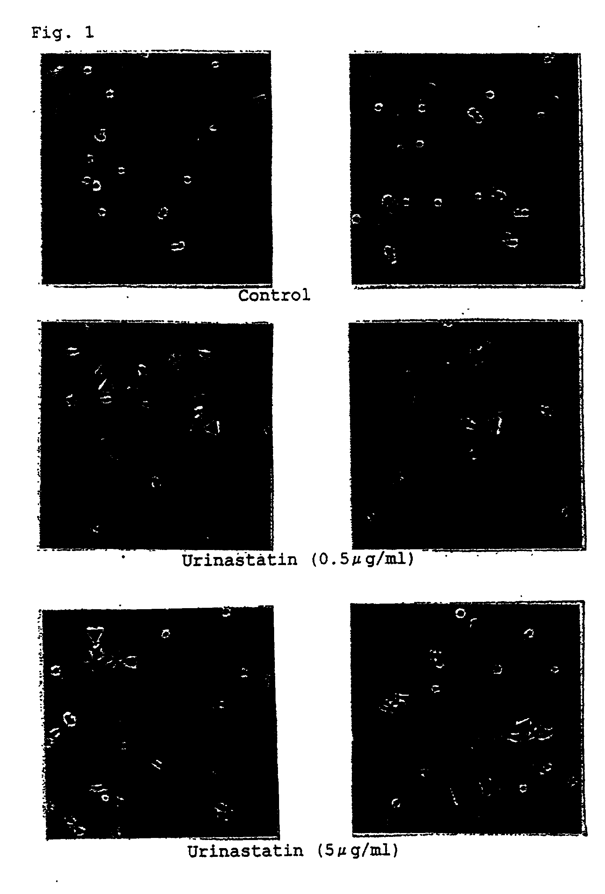

Fig. 1 is a set of photographs showing the adhesion/extension (migration)-promoting action of urinastatin on human corneal epithelial cells.

DISCLOSURE OF THE INVENTION

The present invention is now described in detail.

The first aspect of the present invention is concerned with a therapeutic composition for corneal epithelial disorders which is indicated for the prophylaxis and/or therapy of corneal epithelial disorders, comprising urinastatin as an active ingredient.

Here, the "therapeutic composition for corneal epithelial disorders" means a drug to be used in diseases of the corneal epithelium, and includes a drug for the prophylaxis of corneal epithelial disorders and a therapeutic drug for corneal epithelial disorders. The drug for the prophylaxis of corneal epithelial disorders is a drug to be intended for the prevention of onset of a corneal epithelial disorder in cases where such onset is anticipated, and the therapeutic drug for corneal epithelial disorders is a drug to be used for the cure or remission of corneal epithelial disorders which has already developed.

The term "therapeutic" as used in this specification means that the drug can be administered to an animal inclusive of man for the purpose of therapy, diagnosis and/or prophylaxis of a disease, and the "therapeutic composition" is used herein to mean a therapeutic drug, a diagnostic reagent and/or a prophylactic agent in a broad sense of the term.

The therapeutic composition for corneal epithelial disorders according to the present invention comprises urinastatin as an active ingredient. As used in this specification, the term "urinastatin" means human urinary trypsin inhibitor (UTI).

The urinastatin mentioned above is a glycoprotein having a molecular weight of about 67000 (as measured by gel permeation chromatography) or about 34000 (as measured by SDS-polyacrylamide gel electrophoresis) and is a known substance of which the sugar moiety accounts for about 35% [Journal of Medicine and Pharmaceutical Science, 33, 5, 1089-1097 (1995)]. On the market, urinastatin is available from Mochida Pharmaceutical Co. under the tradename of "Miraclid".

Urinastatin can be purified from fresh healthy male urine by the standard purification procedure, for example by adsorption on diatomaceous earth, silica gel or the like and subsequent treatment with an ion exchange resin, gel filtration, etc. as applied in a serial combination.

With recent advances in analytical techniques, it has become elucidated that urinastatin is composed of several isomers. The difference between those isomers can be characterized by the degree of sulfonation of the sugar moiety. In other respects (antitrypsin activity, molecular weight, amino acid composition, N-terminal amino acid sequence, C-terminal amino acid sequence, sialic acid content, uronic acid content), there seems to be no difference [Yuki, Y. et al.: Biochimica et Biophysica Acta, 1203, 298-303 (1993)]. It is, therefore, obvious that any and all the isomers of urinastatin fall within the purview of the present invention.

With regard to the urinastatin for use in the present invention, its cell growth-stimulating activity has been noted for human fibroblast cells and bovine corneal endothelial cells [Johanna, K. et al.: Biochim. Biophys. Acta, 1221, 145-152 (1994)] but there is no relevant report on human keratoconjunctival epithelial cells and stromal cells. Moreover, the response to a proteinaceous protease inhibitor varies markedly with different cell species and animal species [Mckeehan, W. L., et al.: J. Biol. Chem. 261, 5378-5383 (1986)], so that the pharmacologic effect of this human urinary trypsin inhibitor on human keratoconjunctival epithelial cells could not be predicted.

Japanese Kokai Publication Sho-63-267730 discloses an external drug for the therapy of allergic rhinitis and allergic conjunctivitis, which comprises urinastatin as an active ingredient. It is described that when 20mg/ml was administered to patients with allergic conjunctivitis, the conjunctival injection and itchy sensation disappeared.

On the other hand, the intensive investigations, made by the present inventors in animals about the promoting effect of urinastatin on the adhesion/extension (migration) of corneal epithelial cells, demonstrated, as will be described in detail hereinafter, that corneal epithelial injuries in diabetic rats as well as in rabbits could be dramatically cured, endorsing the physiological activity observed in cell culture. Furthermore, it was found that the above result was attributable to the action to promote the secretion of plasminogen activator, which is necessary for the healing of corneal epithelial wounds, to the extent not far enough to destroy the tissue due to overproduction without inhibiting the plasminogen activator activity. These facts satisfy the requirements of an epochally new therapeutic composition for corneal epithelial disorders, and the first aspect of the present invention is predicated on the above finding.

The therapeutic composition for corneal epithelial disorders according to the present invention can be manufactured by formulating said urinastatin with pharmaceutically acceptable additives.

In the use of said urinastatin as an active ingredient of the therapeutic composition for corneal epithelial disorders in accordance with the present invention, the concentration of urinastatin is not particularly restricted but in light of the results of microscopic and gross observations which verified the promotion of adhesion/extension (migration) of human corneal epithelial cells within the concentration range of 0.05 to 10000 μg/ml, the formulating amount of urinastatin is preferably selected from the above-mentioned range. Furthermore, because a marked proliferation of human corneal epithelial cells was noted at concentrations between 0.5 and 5000 μg/ml, the preferred concentration range of urinastatin is the range mentioned just above.

The therapeutic composition for corneal epithelial disorders according to the present invention is preferably provided in topical dosage forms, particularly in the form of an ophthalmic solution, an ophthalmic ointment or a lyophilized ophthalmic powder, but is not restricted. The ophthalmic solution or ointment, for instance, can be manufactured by using various standard formulating additives, for example preservatives such as sodium dehydroacetate, methyl p-oxybenzoate, etc., isotonizing agents such as sodium chloride, glycerin, etc., thickeners such as carboxymethylcellulose, polyvinyl alcohol, etc., and stabilizers such as sodium edetate, polysaccharides, and so on. The additives are not limited to those mentioned above, however, and may be other substances which are acceptable from the standpoint of eye physiology. The buffer solutions to be added to said various dosage forms are preferably isotonic and non-irritating with the pH 4 to 9, particularly pH 5.5 to 8.0.

Within the range not interfering with the activity of the active ingredient urinastatin, the therapeutic composition for corneal epithelial disorders according to the present invention may contain ophthalmologically bioactive substances such as fibronectin, hyaluronic acid and various glycosaminoglycans (chondroitin sulfate, dermatan sulfate, heparan sulfate, heparin, keratan sulfate), etc. to provide still more potent preparations for corneal epithelial disorders. Furthermore, antibiotics, steroidal antiinflammatory agents such as glucocorticoids, and nonsteroidal antiinflammatory agents may also be formulated.

The administration and dosage of the therapeutic composition for corneal epithelial disorders according to the present invention are dependent on the patient's condition and age, among other factors, but an ophthalmic solution, for instance, can be instilled in the eye usually once ~ 5 times daily, 1 to 5 drops per dose. In the case of an ophthalmic ointment, a suitable amount is applied into the conjuctival saccus usually once ~ 3 times daily.

The corneal epithelial disorders in which the therapeutic composition of the invention can be indicated are not particularly restricted provided that an impairment of the corneal epithelium is involved, thus including keratoconjunctival epithelial disorders associated with endogenous diseases such as herpetic keratitis, bacterial corneal ulcer, neuroparalytic keratitis, diabetic keratopathy, Sjögren's syndrome, Stevens-Johnson syndrome, etc., and exogenous diseases such as postoperative, drug-induced, traumatic and contact lens wearer's diseases.

The second aspect of the present invention is concerned with a therapeutic composition for UV-induced keratoconjunctival epithelial disorders which is indicated for the therapy of UV-induced keratoconjunctival epithelial disorders, comprising urinastatin as an active ingredient.

The term "therapeutic composition for UV-induced keratoconjunctival epithelial disorders" means a drug for disorders of the keratoconjunctival epithelia as caused by ultraviolet radiation and includes, among others, a drug for the prophylaxis of keratoconjunctival epithelial disorders and a therapeutic drug for keratoconjunctival epithelial disorders. The prophylactic drug for keratoconjunctival epithelial disorders is a drug for use in the prevention of the imminent onset of a UV-induced keratoconjunctival epithelial disorder and the therapeutic drug for keratoconjunctival epithelial disorders is a drug for the cure or remission of an UV-induced keratoconjunctival epithelial disorders which has already developed.

Studies by the present inventors revealed that compared with glutathione, vitamin C, vitamin E, lactoferrin or the like, urinastatin has a very effective protective efficacy against damages to the human keratoconjunctival epithelia as caused by ultraviolet radiation. Furthermore, it was confirmed that this protective efficacy is a unique efficacy not shared by any other protease inhibitor. In fact, there has been no report at all on the use of urinastatin in that mode in the field of ophthalmology, and the foregoing facts were unearthed for the first time by the inventors of this invention.

It has been reported that urinastatin has no free radical-scavenging activity, while several reports are available on the free radical-quenching (suppressive) action of urinastatin.

However, as will be shown hereinafter in Examples, the urinastatin content during UV irradiation is extremely low as compared with the hitherto-reported effective amount (37 to 74 µg/ml; 1 to 2 µM, as calculated based on the molecular weight of 34000; the same applies hereinafter) [M. Nomura et al.: Effect of Urinastatin and Aprotin on the oxygen radical production of polymorphonuclear leukocytes; Advances in Medicine, 142, 895-896 (1987)], and if this effect of urinastatin be solely attributed to its free radical-quenching (suppressive) action, a major conflict would be inevitable with the results with glutathione, vitamin C, vitamin E and so on.

Therefore, the protective efficacy of urinastatin against UV damage is not solely attributed to its free radical quenching (suppressive) action but some other factors contributory to the defensive function appears to be involved. Thus, induction of a chaperone or expression of a scavenger receptor protein may for example be postulated.

K. Tsubota et al. contacted human corneal epithelial cells (HCEC) with a high concentration of $H_2O_2$ and demonstrated the production of intracellular OH radicals which is comparable to the response to UV-B irradiation and the consequent reduction in intracellular ATP content. Furthermore, it has been found that UV-B irradiation causes a damage to the inner membrane of mitochondria which is not caused by the OH radical alone [S. Shimmura, et al: Subthreshold UV radiation-induced peroxide formation in cultured corneal epithelial cell: The protective effects of lactoferrin; Exp. Eye Res., 63, 519-526, 1996., K. Tsubota. et al: Ultraviolet B-induced mitochondrial dysfunction is associated with decreased cell detachment of corneal epithelial cell in vitro; Invest. Ophthalmol. Vis. Sci., 38, 620-626, 1997].

Thus, the proliferation of human keratoconjunctival epithelial cells cannot take place unless the UV-B radiation damage to the inner membrane of mitochondria is prevented. Therefore, urinastatin is considered to protect the inner membrane of mitochondria against the damage due to ultraviolet radiation as well.

Thus, UV-induced damage to human keratoconjunctival epithelial cells is remarkably inhibited by urinastatin. And this protective action is by far more potent than the action of antioxidants, such as lactoferrin, glutathione, vitamin E, vitamin C, etc. or the action of protease inhibitors such as $\alpha_1$-trypsin inhibitors, $\alpha_2$-plasmin inhibitors, aprotinin, bestatin and so on.

The mechanism of protective action of urinastatin can hardly be related to any of its quenching (inhibitory) action on the free radicals produced by UV radiation, the action derived from its protease inhibitory activity and the action due to its cell membrane-protecting activity, but it is likely that a mechanism other than those known ones plays a major role.

The indication of urinastatin in UV-induced disorders of the eye has not been contemplated to this day and the second aspect of the present invention is predicated on the above finding.

The therapeutic composition for UV-induced keratoconjunctival epithelial disorders according to the present invention can be manufactured by formulating said urinastatin with pharmaceutically acceptable additives.

The dosage form for the therapeutic composition for UV-induced keratoconjunctival epithelial disorders according to the second aspect of the present invention includes the same dosage forms as mentioned for the therapeutic composition for corneal epithelial disorders according to the first aspect of the present invention, and the concomitant drugs which can be used as well as the administration method and dosage may also be the same as mentioned for the therapeutic composition for corneal epithelial disorders according to the first aspect of the invention.

The keratoconjunctival epithelial disorders in which the therapeutic composition according to the second aspect of the present invention can be indicated include UV-induced conjunctival injection, diffuse superficial keratitis, corneal edema, iritis, etc., actinic keratitis accompanied by necrosis and erosion, "snow blindness" characterized by pain, photophobia and palbebral paralysis, UV-induced cataract in which a plurality of ethiologic factors are involved, immunosuppression resulting from the injury of Langerhans cells, induction of inflammatory cytokines, DNA damage and neuropathy.

The third aspect of the present invention is concerned with a therapeutic composition for keratectomy postoperative corneal epithelial complications which is indicated for the therapy of keratectomy postoperative corneal epithelial complications, comprising urinastatin as an active ingredient.

The term "therapeutic composition for keratectomy postoperative corneal epithelial complications" is used in this specification to mean a drug for disorders of the corneal epithelium which develop postoperatively in keratectomized cases and includes, among others, a prophylactic drug for keratectomy postoperative corneal epithelial complications and a therapeutic drug for keratectomy postoperative corneal epithelial complications. The prophylactic drug for keratectomy postoperative corneal epithelial complications is a drug for prevention of the imminent onset of a corneal epithelial disorder after refractive keratectomy and the therapeutic drug for keratectomy postoperative corneal epithelial complications is a drug for the cure or remission of corneal epithelial disorders developing postoperatively after refractive keratectomy.

The operative procedure for said keratectomy is not particularly restricted but includes, inter alia, photo refractive keratectomy (PRK) which is an operation performed using an excimer laser.

The keratectomy postoperative corneal epithelial complications mentioned above are suspected to result from the exposure of the corneal epithelium to excimer laser light during PRK. The excimer laser beam is an ultraviolet radiation with a wavelength of 193 nm and, therefore, a therapeutic composition capable of preventing or curing the corneal epithelial disorders caused by the ultraviolet rays with a wavelength of 193 nm is suited for the above-mentioned purpose.

The inventors of the present invention found after much research that urinastatin is very efficacious for the above purpose, and perfected the present invention.

The use of urinastatin in keratectomy postoperative disorders of the corneal epithelium for the above-mentioned purpose has not been contemplated to this day and the third aspect of the present invention is predicated on this new knowledge.

The therapeutic composition for keratectomy postoperative corneal epithelial complications according to the present invention can be manufactured by formulating said urinastatin with pharmaceutically acceptable additives.

The dosage form for the therapeutic composition for keratectomy postoperative corneal epithelial complications according to the third aspect of the present invention includes the same dosage forms as mentioned for the therapeutic composition for corneal epithelial disorders according to the first aspect of the present invention, and the concomitant drugs which can be used as well as the administration method and dosage may also be the same as mentioned for the therapeutic composition for corneal epithelial disorders according to the first aspect of the invention.

The corneal epithelial complications in which the therapeutic composition according to the third aspect of the present invention is to be indicated includes, inter alia, those affecting the various laminae of the cornea, epithelial defect, subepithelial haze, and, among damages to the endothelium, decrease in the number of endothelial cells; as examples of those affecting the entire layer of the cornea, corneal perforation and corneal infections; and as examples of those affecting the visual function, halo, glare, undercorrection, overcorrection and regression, among others.

The fourth aspect of the present invention relates to a therapeutic composition for drug-induced keratoconjunctival epithelial disorders which is indicated for the therapy of drug-induced keratoconjunctival epithelial disorders caused by the use of ophthalmic drugs, comprising urinastatin as an active ingredient.

The therapeutic composition for drug-induced keratoconjunctival epithelial disorders is a therapeutic composition used for the therapy or alleviation of various keratoconjunctival epithelial disorders caused by ophthalmic drugs.

The inventors of the present invention discovered after intensive research that urinastatin is very effective against various keratoconjunctival epithelial disorders induced by ophthalmic drugs and have perfected the present invention.

The ophthalmic drugs mentioned above are not particularly restricted as far as they have the risk for inducing onset of a keratoconjunctival epithelial disorder upon administration and include the following drugs, among others.

Oral ophthalmic drugs: dichlorphenamide, helenien, calcium diiodostearate, methazolamide, concentrated glycerin;

Miotics: pilocarpine hydrochloride, physostigmine salicylate, p-nitrophenyl-ethyl ethylphosphate, ecothiopate iodide, distigmine bromide, carbachol;

Antiglaucoma drugs: timolol maleate, befunolol hydrochloride, carteolol hydrochloride, unoprostone isopropyl ester, latanoprost;

Mydriatics: cyclopentorate hydrochloride, homatropine hydrobromide, atropine sulfate, phenylephrine hydrochloride, tropicamide, epinephrine, epinephrine bitartrate, dipivefrin hydrochloride;

Anticataract drugs: pirenoxine, Phacolysin (TM, Zeria Pharmaceutical), glutathione;

Corneal protectant/therapeutic drugs: sodium chondroitin sulfate, Flavitan (TM, Yamanouchi Pharmaceutical), cyanocobalamin, dipotassium glycyrrhizinate, sodium hyaluronate;

Enzymes: α-chymotrypsin;

Vasodilators: naphazoline nitrate;

Antiseptic/astringents: silver nitrate, boric acid, borax;

Antibiotics, antifungal agents, synthetic antibacterial agents, sulfa drugs, antiviral agents;

Adrenocortical hormones: hydrocortisone acetate, prednisolone acetate, dexamethasone sodium phosphate, dexamethasone, betamethasone sodium phosphate, fluorometholone;

Others: zinc sulfate, sodium azulene sulfonate, lysozyme chloride, Tego-51, polyvinyl alcohol;

Nonsteroidal antiinflammatory agents: diclofenac sodium, indomethacin, pranoprofen;

Antiallergic agents: chlorpheniramine maleate, sodium cromoglycate, amlexanox, ketotifen fumarate, tranilast;

Local anesthetics: oxytetracaine hydrochloride, T-caine.

The fourth aspect of the present invention enables effective indication of ophthalmic drugs through quenching the side effects of in keratoconjunctival epithelial disorders induced by said drugs.

It was not known that urinastatin could be utilized as a therapeutic composition for drug-induced keratoconjunctival epithelial disorders as caused by ophthalmic drugs, and the fourth aspect of the present invention is predicated on the above finding.

The therapeutic composition for drug-induced keratoconjunctival epithelial disorders according to the present invention can be manufactured by formulating said urinastatin with pharmaceutically acceptable additives.

The dosage form for the therapeutic composition for drug-induced keratoconjunctival epithelial disorders according to the fourth aspect of the present invention includes the same dosage forms as mentioned for the therapeutic composition for corneal epithelial disorders according to the first aspect of the present invention, and the concomitant drugs which can be used as well as the administration method and dosage may also be the same as mentioned for the therapeutic composition for corneal epithelial disorders according to the first aspect of the invention.

The keratoconjunctival epithelial disorder, in which this therapeutic composition for drug-induced keratoconjunctival epithelial disorders according to the fourth aspect of the present invention can be indicated, includes not only the disorders mentioned hereinbefore for the therapeutic composition for corneal epithelial disorders according to the first aspect of the present invention but also the keratoconjunctival epithelial disorders resulting from administration of ophthalmic drugs.

The fifth aspect of the present invention relates to a therapeutic composition for dry eye which is indicated for the prophylaxis and/or therapy of dry eye, comprising urinastatin as an active ingredient.

The term "therapeutic composition for dry eye" means a drug to be indicated in dry eye, and includes a prophylactic drug for dry eye and a therapeutic drug for dry eye. The prophylactic drug for dry eye is a drug to be used for preventing the predicted onset of dry eye and the therapeutic drug for dry eye is a drug to be used for the cure or remission of a dry eye.

As will be described in detail hereinafter, it has been confirmed that urinastatin certainly prevents dry eye in an animal model and further that this result is attributable to the fact that urinastatin promotes the mucin 1 production of keratoconjunctival epithelial cells. These facts satisfy the epochaly new requirements of a therapeutic composition for dry eye and this aspect of the present invention is predicated on the above finding.

The therapeutic composition for dry eye according to the present invention can be manufactured by formulating urinastatin with pharmaceutically acceptable additives.

In the use of urinastatin as the active ingredient of said therapeutic composition for dry eye, the concentration range of urinastatin which can be used is not particularly restricted. However, in view of the finding that it promotes the mucin 1 production of human keratoconjunctival epithelial cells at 0.05 to 10000 μg/ml and that urinastatin could actually prevent dry eye in a rat model at the same concentration, the concentration of urinastatin is more preferably within the above range. Furthermore, since the mucin production of human keratoconjunctival epithelial cells was particularly high when the concentration of urinastatin was 0.5 to 5000 μg/ml, it is more preferable to formulate urinastatin within the concentration range mentioned just above.

The dosage form for the therapeutic composition for dry eye according to the fifth aspect of the present invention includes the same dosage forms as mentioned for the therapeutic composition for corneal epithelial disorders according to the first aspect of the present invention, and the concomitant drugs which can be used as well as the administration method and dosage may also be the same as mentioned for the therapeutic composition for corneal epithelial disorders according to the first aspect of the invention.

The indications for the therapeutic composition for dry eye according to this invention include various forms of dry eye in which a quantitative decrease or qualitative abnormality of tears is present regardless of the presence or absence of keratoconjunctival impairment, such as hypolacrimia, xerophthalmia, Sjögren's syndrome, Stevens-Johnson syndrome, pemphigus, disease of lid margin, lid closure insufficiency, sensory nerve palsy, etc.

The above-mentioned therapeutic composition for corneal epithelial disorders, therapeutic composition for UV-induced keratoconjunctival epithelial disorders, therapeutic composition for keratectomy postoperative corneal complications, therapeutic composition for drug-induced keratoconjunctival epithelial disorders, and therapeutic composition for dry eye, all of which are provided by the present invention, can be administered as ophthalmic drugs to animals inclusive of man, for example by instillation into the eye. The treating method which comprises applying any of said ophthalmic drugs of the present invention to animals inclusive of man for therapeutic and/or prophylactic purposes is also subsumed in the category of the present invention.

The use of urinastatin of the invention for the manufacturing of said ophthalmic drugs of the present invention also falls within the purview of the present invention.

The therapeutic composition for corneal epithelial disorders, therapeutic composition for UV-induced keratoconjunctival epithelial disorders, therapeutic composition for keratectomy postoperative corneal complications, therapeutic composition for drug-induced keratoconjunctival epithelial disorders, and therapeutic composition for dry eye, all of which are provided by the present invention, are ophthalmic compositions comprising urinastatin as the active ingredient and any such composition falls within the purview of the present invention as far as it contains urinastatin as an active ingredient, regardless of whether it additionally contains other ingredients, as mentioned hereinbefore.

The therapeutic composition for corneal epithelial disorders, therapeutic composition for UV-induced keratoconjunctival epithelial disorders, therapeutic composition for keratectomy postoperative corneal complications, therapeutic composition for drug-induced keratoconjunctival epithelial disorders, and therapeutic composition for dry eye, all of which are provided by the present invention, are pharmaceutical compositions which can be administered to animals inclusive of man and, therefore, can be also called the pharmaceutical composition for corneal epithelial disorders, pharmaceutical composition for UV-induced keratoconjunctival epithelial disorders, pharmaceutical composition for keratectomy postoperative corneal complications, therapeutic composition for drug-induced keratoconjunctival epithelial disorders, and pharmaceutical composition for dry eye according to the present invention.

BEST MODE FOR CARRYING OUT THE INVENTION

The following reference and working examples illustrate the present invention in further detail without delimiting its scope in any manner.

Reference Example 1

Production of urinastatin

About 1000 L of healthy adult human urine was adjusted to pH 10.0 with 6 N-NaOH and allowed to stand for about 30 minutes. The insoluble matter formed was removed with a Tetron cotton column and the column effluent was adjusted to pH 7.0 with 6 N-$H_2SO_4$. Air was bubbled through 990 L of the liquid for foaming and 140 L of the foam fraction was harvested.

The harvested foam fraction was adjusted to pH 5.0 with 6 N-HCl and adsorbed on 100 ml of silica gel 5D (Fuji-Davison) batchwise at about 10 °C for 4 hours.

The silica gel 5D was packed into a column and washed with an aqueous 0.05 N-sodium dihydrogenphosphate-0.5 M NaCl solution (pH 5.0), and urinastatin was eluted with 0.3 M ammonium chloride-aqueous ammonia (pH 9.5). The eluate was subjected to 70% ammonium sulfate precipitation at room temperature and after 24 hours' standing, the precipitate was recovered. This precipitate was dissolved in an aqueous solution of sodium dihydrogenphosphate (pH 4.0) and run onto a DEAE-Cellulofine A200 column (resin content 300 ml) equilibrated with 0.1 M NaCl-containing aqueous solution of sodium dihydrogenphosphate (pH 4.0). After the column was washed with the same buffer as above, urinastatin was eluted with a 0.3 M NaCl-containing aqueous solution of sodium dihydrogenphosphate (pH 4.0). The eluate was further diluted with pure water and subjected to rechromatography under the same conditions as above. The eluate was brought back to pH 7.0 with 0.3 N-NaOH and passed through an aprotinin-Sepharose CL-4B column to remove traces of contaminant kallikrein. The effluent was filtered through PM-100 (Amicon) ultrafiltration membrane and the filtrate was further concentrated with PM-10 (Amicon) ultrafiltration membrane and finally filtered through a 0.22 μm membrane filter to recover $6\times10^5$ units of purified urinastatin having a specific activity of 2700 U/mg protein (Lot A).

By the same purification procedure, an additional 3 lots of purified urinastatin were prepared (Lot B: specific activity 2800 U/mg protein, yield $5.9\times10^5$ U; Lot PW-2: specific activity 2800 U/mg protein, yield $5.65\times10^5$ units; Lot PW-3: specific activity 2600 U/mg protein, yield $6.5\times10^5$ U).

By the same purification procedure as above, a further purified urinastatin (Lot 67B8: specific activity 2740 U/mg protein, yield $6\times10^5$ U) was obtained.

This purified urinastatin gave a single band at the molecular weight of 34000 on an SDS electrophoretogram (12% gel), which formed a precipitation line with the rabbit antibody prepared using urinastatin standard as an antigen in the gel double diffusion assay and passed the biological specification tests such as pyrogen test and thromboplastin test.

Example 1

Adhesion and extension (migration) of human corneal epithelial cells

A cell suspension flask (Sumitomo Bakelite) was seeded with 300 μl of a culture fluid obtained by suspending human corneal epithelial cell line HCEC in a serum-free medium (0.1 mg/ml kanamycin sulfate-containing D-MEM/F-12 medium; Nikken Biomedical Research Institute) and adjusted to a cell population of $2.0\times10^5$/ml and, at the same time, urinastatin (Lot PW-2) was added in a varying concentration. After 5 hours of culture in a 5% $CO_2$ incubator at 37°C, the medium was thoroughly removed and 300 μl of the same fresh serum-free medium as above was added to each well, followed immediately by photographing under microscopic observation to evaluate the degree of adhesion and extension (migration) of the human corneal epithelial cells. In the control cell group, saline in lieu of urinastatin was added.

The results are shown in Fig. 1. In Fig. 1, the two photographs in the top row represent the control group, the two photographs in the middle row represent the urinastatin (concentration 0.5 μg/ml)-added group, and the two photographs in the bottom row represent the urinastatin (concentration 5 μg/ml)-added group. It will be apparent from Fig. 1 that, compared with the control, a considerably large number of cell clusters (indicated by arrowhead) indicative of adhesion and extension (migration) of human corneal epithelial cells was observed in the multiwells to which urinastatin had been added.

Example 2

Proliferation of human corneal epithelial cells

A 96-well multiwell plate (Corning) was seeded with 100 μl of a cell suspension prepared by suspending human corneal epithelial cell line HCEC in 1% fetal bovine serum-SHEM (supplementary hormone epithelial medium) modified medium (the SHEM medium described in Araki, K. et al.: Invest. Ophthalmol. Vis. Sci., 18, 2665, 1993, from which epidermal growth factor was omitted) and adjusted to a concentration of $3\times10^4$ cells/ml, and almost immediately 10 μl/well of urinastatin was added. The cells were cultured in a 5% $CO_2$ incubator at 37 °C for 48 hours. Two lots of urinastatin (Lot A: 1.6 and 0.16 mg/ml, Lot B: 2.2 mg/ml) were used. After 48 hours of culture, 10 μl of a 5 mg/ml solution of MTT (3-(4,5-dimethyl-2-thiazolyl)-2,5-diphenyl-2H-tetrazolium bromide) was added to each well and the culture was continued in a 5% $CO_2$ incubator at 37 °C for 16 hours. Then, 100 μl of 20% SDS (sodium dodecyl sulfate)-0.01 N-HCl was added to each well to stop cell culture and the system was further incubated at 37°C for 6 hours. The amount of blue formazan produced was calculated from the absorbance at 570 nm ~ 660 nm as measured with a microplate reader to quantitate the proliferation of human epithelial cells. The cell proliferation promoting activity of urinastatin was estimated using the cell group treated with 10 μl of saline in lieu of urinastatin as control and expressed in (absorbance in urinastatin-treated cell group ÷ absorbance in saline-treated cell group) × 100 (%). The urinastatins used varied in the proportions of isomers as shown in Table 1. The results are shown in Table 2. It will be apparent from Table 2 that although the promoting effect of urinastatin on the proliferation of human epithelial cells varied with different proportions of isomers, proliferations more than about twice the control were obtained.

Table 1

| Proportions of urinastatin isomers | | | | |
|---|---|---|---|---|
| | Isomers (%) | | | |
| | U-1 | U-2 | U-3 | U-4 |
| Lot. A | 2.4 | 32.1 | 51.3 | 14.2 |
| Lot. B | 16.4 | 37.6 | 51.7 | 14.3 |

Table 2

| Efficacy of urinastatin on the proliferation of human corneal epithelial cells | |
|---|---|
| Urinastatin (final concentration) | Proliferation rate (%) |
| Lot. A (160 μg/ml) | 194±20 |
| Lot. B (220 μg/ml) | 245±5.1 |

Example 3

Proliferation of human corneal epithelial cells

A 48-well multiwell plate (Corning) was seeded with 300 μl of a cell suspension prepared by suspending human corneal epithelial line HCEC in 1% fetal bovine serum-SHEM modified medium at a concentration of $5 \times 10^4$ cell/ml and, at the same time, 30 μl/well of a varying concentration of urinastatin (Lot PW-2, PW-3) was added at final concentrations of 0.05 to 1 μg/ml. The cells were cultured in a 5% $CO_2$ incubator at 37 °C for 64 hours.

After completion of culture, the culture supernatant was removed from each well and after addition of 100 μl of a detaching solution (phosphate-buffered saline containing 0.25% (w/v) trypsin and 0.02% EDTA), the system was further incubated in the 5% $CO_2$ incubator at 37 °C for 6 minutes.

After incubation, the epithelial cells were further detached and dispersed by pipetting. The cells were stained by adding 100 μl of 0.1% (w/v) trypan blue solution and immediately counted with a hemocytometer. A cell group in which 30 μl of saline was added in lieu of urinastatin was established as control.

The results are shown in Table 3. The data in Table 3 indicate a concentration-dependent definite promoting effect of urinastatin on the proliferation of human corneal epithelial cells.

Table 3

| Efficacy of urinastatin favoring the proliferation of human corneal epithelial cells | |
|---|---|
| Concentration of urinastatin (μg/ml) | Cell count ($\times 10^5$ cells/ml) |
| Control | 2.40 |

Table 3 (continued)

| Efficacy of urinastatin favoring the proliferation of human corneal epithelial cells | | |
|---|---|---|
| Concentration of urinastatin (µg/ml) | | Cell count ($\times 10^5$ cells/ml) |
| PW-2 | 0.05 | 2.23 |
| | 0.1 | 2.50 |
| | 0.2 | 2.53 |
| | 0.5 | 2.90 |
| | 1 | 3.43 |
| PW-3 | 0.05 | 2.43 |
| | 0.1 | 2.75 |
| | 0.2 | 2.75 |
| | 0.5 | 2.93 |
| | 1 | 3.00 |

Example 4

Therapy of corneal epithelial disorders

The corneal reepithelialization-promoting activity was evaluated in diabetic rats with deepithelialized cornea.

Eight-week-old male Sprague-Dawley rats were fasted overnight and a solution of streptozocin (Sigma) in 3 mM citrate buffer (pH 4.5) was administered from the tail vein in a dose of 60 mg/kg body weight. The animals were further fed for 2 weeks to construct diabetic rats.

The diabetic rats thus prepared were divided into 4 groups, and each animal was subjected to general anesthesia with 40 mg/kg body weight of Nembutal Injection (Dainippon Pharmaceutical) i.p. and local anesthesia by instilling one drop/eye of 0.4% solution of Benoxil (Santen Pharmaceutical). Then, using an ophthalmic knife, the whole corneal epithelium was scraped off from the limbus to the center of the cornea. Starting immediately after total corneal epithelial excision, 5 µl each of urinastatin (Lot PW-2) 5 µg/ml or 500 µg/ml, Hyalein-Mini 0.3 (Santen Pharmaceutical), or saline (Hikari Pharmaceutical) was topically administered to rats in the corresponding groups, 6 times daily at 2-hour intervals.

Furthermore, immediately after said total corneal epithelial excision and at 24 hours and 32 hours after excision, one drop of Richardson stain (a 1:1 mixture of 1% methylene blue and 1% azure II in a 1% aqueous solution of sodium borate) was instilled into the eye. After thorough washing with saline (Hikari Pharmaceutical), the lesion was stained and the anterior segments of bilateral eyes were photographed under a stereoscopic microscope carrying a video camera. The area of corneal epithelial wound was determined from the image of the stained area on the monitor screen using an image analyzing system software (NIH Image 1.61). With the wound area immediately after epithelial excision being taken as 100%, the degree of corneal reepithelialization was expressed as reductions in wound area and shown in Fig. 4. The animal model used in this example was more refractory to treatment than the animal model used in Example 6 given hereinafter because the staining solution used was Richardson stain which is known to delay corneal epithelial wound healing [Ubels, J. et al.,: Invest. Ophthalmol. Vis. Sci., 23, 127 (1982)] and substantially no healing occurred in the control group even after 32 hours of epithelial excision. Substantially no healing was found, either, in the group treated with Hyalein-Mini 0.3 (the active ingredient sodium hyaluronate 0.3%) which is a topical ophthalmic solution for the therapy of corneal epithelial disorders. In contrast, significant reductions in corneal wound area were observed in the urinastatin instillation group. It is, therefore, clear that urinastatin significantly promotes the healing of refractory corneal epithelial wounds.

Table 4

| Efficacy of urinastatin in diabetic rat corneal epithelial impairment | | | | |
|---|---|---|---|---|
| | Control | Urinastatin (500 μg/ml) | Urinastatin (5 μg/ml) | Hyalein®-Mini 0.3 |
| 0 Hr | 100 | 100 | 100 | 100 |
| 24 Hr | 97.9±11.7 | 87.9±8.8 | 89.2±9.2 | 107.3±3.7 |
| 32 Hr | 102.1±15.0 | 66.4±8.4** | 72.8±22.5* | 96.6±11.6 |
| The corneal lesion area immediately after excision of the corneal epithelium (0 hr) is taken as 100%. | | | | |

*p<0.05,
**p<0.01

Example 5

The influence of urinastatin and aprotinin on human corneal epithelial cell plasminogen activator (PA)

The proposition that the PA secreted by corneal epithelial cells plays an important role in the process of recovery of corneal epithelial cells injured by various causes was advanced by Thoft R. A. et al., as early as in 1983 [Thoft, R. A. et al.: Hypothesis of corneal epithelial maintenance, Invest. Ophthalmol. Vis. Sci., 24, 1442-1443 (1983)].

On the other hand, it was reported in 1987 by Salonen E. M. et al. that aprotinin which inhibits plasmin activity is instrumental to wound healing, that is to say indirect inhibition of PA activity is important [Salonen, E. M. et al.: Plasmin in tear fluid of patients with corneal ulcers: basis for new therapy, Acta Ophthalmol., 65, 3-12 (1987); Cejkova, J. et al.: Histochemical study of alkali-burned rabbit anterior eye segment in which severe lesions were prevented by aprotinin treatment, Histochemistry, 92, 441-448 (1989)].

However, in 1991 James, D. et al. reported that the movement of the corneal epithelium which is necessary for the process of corneal epithelial wound healing is inhibited concentration-dependently by aprotinin [James, D. et al.: Effect of protease inhibitors on corneal epithelial migration., Invest. Ophthalmol. Vis. Sci., 32, 2073-2078 (1991)]

Furthermore, in 1998 Winston, W. Y. K. et al. reported a decisive finding. They constructed an animal model of mechanical corneal epithelial wound using plasminogen-defective mice and observed the course of wound healing. As a result, serious and persistent inflammatory reactions, fibrin deposits posteriorly of the corneal epithelium, ulceration, and complicated malignancies such as stromal neovascularization were observed. They accordingly concluded that the production of plasmin is essential to corneal epithelial wound healing [Winston, W. Y. K. et al.: Healing of corneal epithelial defects in plasminogen and fibrinogen deficient mice., Invest. Ophthalmol. Vis. Sci., 39, 502-508 (1998)].

This finding endorses the absolute necessity of PA in charge of plasmin production. More recently, Thomas, D. et al. conducted a corneal epithelial cytotoxicity test using PA as a marker. Thus, they suggested that whereas a drug which inhibits PA secretion has cytotoxicity and unsuitable for wound prevention, a drug which promotes PA secretion is useful for protection against corneal epithelial wounds during surgery [Thomas, D. et al.: Cytotoxicity of viscoelastics on cultured corneal epithelial cells measured by plasminogen activator release., J. Refract. Corneal Surg., 10, 95-102 (1994)].

It has been reported by many workers that the secretion of PA and maintenance of its activity are essential to corneal epithelial wound healing [Andras, B. et al.: Tear plasminogen activators-indicators of epithelial cell destruction. The effect of excision, n-heptanol debridement, and alkali burn of the cornea on the plasminogen activator activity of rabbit tears., Inter. Ophthalmol., 15, 363-369 (1991); Chris, P. L.: Plasmin and plasminogen activator inhibitors after excimer laser photorefractive keratectomy: New concept in prevention of postoperative myopic regression and haze., Refract. & Corneal Surg., 9, 300-302 (1993)].

Furthermore, Jozsef, T. et al. found high levels of PA inhibitors (PAI) in tears in various keratoconjunctival diseases and Shögren's and reported decreases in PA activity [Jozsef, T. et al.: Plasminogen activator inhibitors in human tears., Acta Ophthalmol., 69, 426-431 (1991)].

It was concluded from those reports of many workers that in discussions about the merit and demerit of the plasminogen activator-plasmin system in corneal epithelial wound healing, the secretion of PA and maintenance of its activity are essential conditions.

Therefore, it was explored in the following example whether urinastatin has demerits similar to those of aprotinin.

Example 5-1

The promoting action of urinastatin on human corneal epithelial cell PA secretion

A 24-well multiwell plate (Corning) was seeded with 500 μl/well of a cell suspension prepared by dispersing human corneal epithelial cell line HCEC in 10% fetal bovine serum-SHEM modified medium to a cell population of $1\times10^4$/ml and the cells were precultured in a 5% $CO_2$ incubator at 37°C for 3 hours. Then, 10 μl/well of urinastatin (Lot PW-2, 50 μg/ml) was added and the cells were cultured under the same conditions as above for 3 days or 6 days, after which time the culture supernatants were respectively harvested. Each of the harvested supernatants was concentrated to about 1/5 with a centrifugal separation membrane (Ultracent; Tosoh Corporation) and the PA activity was determined by the fibrin plate assay which is a partial modification of the method of Ploug, J. et al. [Ploug, J. et al.: Biochim. Biophys. Acta., 24, 278 (1957)] and the synthetic substrate 3145-V method (Peptide Research Institute) [Kawabata, S. et al.: Eur. J. Biochem., 172, 17 (1988)]. The identification of PA was made by confirming the cross reaction of urokinase antibody (TechnoClone G. m. b. H., Austria) and tissue plasminogen activator antibody (Accurate Chemical & Scientific Corporation, U.S.A.) by the Ouchterlony method (gel double diffusion method). The results are shown in Table 5.
In the 3-day culture, no difference was found between the urinastatin group and the urinastatin-free group. In the 6-day culture, however, a definite tendency toward increased PA secretion was found in the urinastatin group compared with the urinastatin-free group. This finding indicates that urinastatin promotes secretion of PA, which is essential to corneal epithelial wound healing, slightly to an extent short of causing tissue destruction due to overproduction so as to effectively realize the wound healing.

Table 5

| Secretogogic effect of urinastatin on human corneal epithelial cell plasminogen activator | | |
|---|---|---|
| Culture time, in days | Plasminogen activator activity (mU/ml) | |
| | Without urinastatin | With urinastatin |
| After 3 days | 1500±44 | 1700±49 |
| After 6 days | 1500±29 | 1900±110 |

Example 5-2

Comparison of urinastatin with aprotinin in the inhibitory effect on human corneal epithelial cell PA activity

Human corneal epithelial cell line HCEC was cultured in 10% fetal bovine serum-containing SHEM modified medium in a 5% $CO_2$ incubator at 37 °C for 6 days and 490 ml of the culture supernatant was subjected to 60% saturation ammonium sulfate precipitation. The resulting precipitate was centrifugally recovered and dissolved in 25 ml of 50 mM phosphate buffer (pH 7.0). This solution was loaded onto a zinc chelate Sepharose CL-6B column (Amersham Pharmacia Biotech) equilibrated with 50 mM phosphate buffer (pH 7.0) and after the column was washed with the same buffer thoroughly, PA was eluted with the same buffer containing 0.3 M sodium chloride and 0.1 M imidazole. The eluate, 17 ml, was concentrated with PM-10 (Amicon) and 3 ml of the concentrate was subjected to gel permeation chromatography using a Sephacryl S200 (30 ml) column (Amersham Pharmaceutical Biotech) to give a partially purified PA sample with a specific activity of 35000 U/protein. This partially purified PA sample (10 U/ml), 50 μl, was added to 0.5 ml of 50 mM NaCl-containing 0.1 M Tris-HCl buffer (pH 8.0), followed by addition of urinastatin (1700 U/ml) and aprotinin (1700 U/ml; Bayer), respectively, to the test tubes. The tubes were incubated at 35°C for 3 minutes. Then, 0.5ml of 25 μM synthetic substrate 3145-V was added to both test tubes and the tubes were incubated at 35 °C for 30 minutes. After this incubation time, the reaction was stopped with 2 ml of 20% acetic acid/$H_2O$. The peptidolysis of synthetic substrate 3145-V by PA was quantitated with a fluorescent spectrophotometer (exciting wavelength 380 nm, fluorescent emission wavelength 460 nm) and the percent inhibition of PA activity was calculated for urinastatin and aprotinin, respectively, by means of the following equation.

Percent inhibition of PA activity = (1 - residual PA activity in inhibitor-added group/PA activity in inhibitor-free group) × 100

The results are shown in Table 6. It can be seen from Table 6 that the inhibition of PA activity by urinastatin was 3% at most and substantially negligible. It is reported that the inhibition of plasmin is also weak [50% inhibition = 830 U/ml; Haruo Ohnishi et al.: Folia Pharmacologica Japonica, 81, 235-244, 1983]. On the other hand, aprotinin shows an inhibition of not less than 30% and can be regarded as an inhibitor of plasminogen-to-plasmin conversion. Furthermore, the above literature reports that aprotinin inhibits plasmin strongly as well (50% inhibition = 6.4 U/ml). Therefore, it is quite likely that aprotinin will completely inhibit the processes related to the plasminogen activator-plasmin system in the first phase of corneal epithelial wound healing. It is, thus, clear that, as a therapeutic drug for corneal epithelial disorders, urinastatin is definitely more efficacious than aprotinin.

Table 6

| Relative inhibition of human corneal epithelial cell plasminogen activator activity by urinastatin and aprotinin | |
| --- | --- |
| | Inhibition rate |
| Urinastatin (1700 U/ml) | 3% |
| Aprotinin (1700 U/ml) | 32% |

Example 6

Therapeutic effect of urinastatin on diabetic rat corneal  epithelial lesion

Male SD rats (7 weeks, old; purchased from Charles River, Japan) were acclimatized for 1 week after arrival. At the age of 8 weeks, streptozotocin (STZ; Sigma) dissolved in 3 mM citrate buffer (pH 4.5) was administered from the tail vein (60 mg/kg) and the rats were further reared for 2 weeks to induce onset of diabetes. On day 11 after administration of STZ, the glucose in the rat urine was assayed with Pretest 3a (Wako Pure Chemical) to confirm the establishment of a diabetic model. Two weeks after STZ administration, Nembutal Injection (Dainabot) was administered intraperitoneally (40 mg/kg) for general anesthesia and, further, one drop of the ophthalmic topical anesthetic Benoxil 0.4% (Santen Pharmaceutical) was instilled into the eye. Then, the epithelial layer of the cornea was detached from the limbus to the center of the cornea. Immediately after total epithelial excision, one drop of 1% (w/v) fluorescein sodium/$H_2O$ was instilled. After the eye was washed with saline, the stained lesion was confirmed under the stereoscopic microscope and recorded with a video camera. Starting immediately after total corneal epithelial excision, the test substances, i.e. urinastatin (Lot PW-2; 50 to 500 μg/ml), rat serum, and Hyalein-Mini (Santen Pharmaceutical), were respectively instilled, 5 μl/eye per dose, 6 times daily at 2-hour (approx.) intervals, using both eyes of 4 animals per group. Saline was used as control. The video recording of the fluorescein Na-stained wound area was performed immediately after corneal epithelial excision and, thereafter, at 8 hr, 24 hr, 32 hr, 48 hr and 72 hr, or a total of 6 times, and using Olympus Video Micrometer VM-50 (NIH Image 1.61), the planimetry of the wounded area was carried out on each occasion.

The effect on corneal epithelial wound healing was evaluated from the serially measured area values using the wound area value immediately after corneal epithelial excision as 100%. The results are shown in Table 7. It is apparent from Table 7 that urinastatin is remarkably superior to the reference drugs in the therapeutic effect on corneal epithelial wound healing.

Table 7

| | Control | Urinastatin (500 µg/ml) | Urinastatin (50 µg/ml) | Rat serum (3-fold dilution) | Hyalein-Mini 0.3 |
|---|---|---|---|---|---|
| Therapeutic efficacy of urinastatin in the diabetic rat corneal lesion model | | | | | |
| 0 Hr | 100% | 100% | 100% | 100% | 100% |
| 8 Hr | 100±20.7 | 91.5±19.2* | 103.2±7.0 | 100±8.0 | 89.9±3.7 |
| 24 Hr | 78.9±12.4 | 55.0±13.5** | 63.6±13.3 | 65.7±+6.2 | 64.1±10.5 |
| 32 Hr | 51.5±6.6 | 31.1±13.0** | 43.3±12.2 | 51.4±12.5 | 48.8±12.1 |
| 48 Hr | 13.7±4.4 | 5.2±5.9* | 14.3±10.8 | 17.3±12.3 | 15.6±6.8 |
| 72 Hr | 0 | 0.1±0.2 | 2.0±3.9 | 3.8±6.2 | 0.4±0.6 |
| The corneal lesion area immediately after excision of the corneal epithelium (0 hr) is taken as 100%. | | | | | |

*p<0.05,
**p<0.01

Example 7

Effect of urinastatin on UV-induced human corneal epithelial cell damage

Human corneal epithelial cell line HCEC was suspended in 10% fetal bovine serum-SHEM modified medium at a concentration of $1\times10^5$ cells/ml and 500 µl of this cell suspension was seeded into each well of a 24-multiwell plate (Corning). The cells were cultured in a 5% $CO_2$ incubator at 37°C for 16 to 17 hours. Then, the culture supernatant was thoroughly removed and 500 µl of 10% fetal bovine serum-SHEM modified medium containing a test sample was added to each well. The cells were cultured under the same conditions as above for a further two hours. After completion of culture in contact with the sample, the culture supernatant was removed thoroughly so that there would be substantially no residue of the sample in the multiwell plate.

The multiwell plate in this condition was irradiated with UV-C, the ultraviolet light with a maximum lethal power, at a distance of 20 to 30 cm for 5 seconds. After UV-C irradiation, 500 µl/well of 10% fetal bovine serum-SHEM modified medium containing the same sample as above was added again and the cells were cultured under the same conditions as above for 16 to 17 hours. Then, the supernatant was discarded from each well and 100 µl of an excision solution (PBS containing 0.25% trypsin (w/v) and 0.02% EDTA) was added. The plate was then incubated in a 5% $CO_2$ incubator at 37 °C for 6 minutes. After this incubation, the viable epithelial cells were thoroughly detached and dispersed by pipetting and stained with 100 µl of 0.1% (w/v) trypan blue staining solution, and immediately the viable cells were counted with a hemocytometer. The protective effect of the sample against UV-induced human corneal epithelial cell damage was evaluated by the following computation.

$$\text{Protective effect (\%)} = (\text{number of viable cells in sample-added group} \div$$
$$\text{number of viable cells in sample-free group}) \times 100$$

The results are shown in Tables 8-1 and 8-2.

Table 8-1

| Efficacy of urinastatin in UV-induced human corneal epithelial cell damage | |
| --- | --- |
| Sample (concentration) | Protective effect (%) |
| Urinastatin (0.3 µM) | 155±7.0** |
| Urinastatin (3 µM) | 211±8.3** |
| Aprotinin (0.3 µM) | 86.6±13 |
| Aprotinin (3 µM) | 77.2±5.1 |

**$p < 0.01$

Table 8-2

| Efficacy of urinastatin in UV-induced human corneal epithelial cell damage | |
| --- | --- |
| Sample (concentration) | Protective effect (%) |
| Urinastatin (0.3 µM) | 140 |
| Human lactoferrin (2 mM), Sigma | 101 |
| Deferoxamine mesylate (2 mM), Ciba-Geigy Japan | 94 |
| Vitamin E (DL-α-tocopherol acetate, 20 µM), Kanto Chemical | 107 |
| Glutathione (2 mM), Sigma | 95 |
| Cysteine (2 mM), Wako Pure Chemical | 46 |
| Vitamin C (ascorbic acid, 2 mM), Roche Japan | 104 |

It can be seen from Table 8-1 that the cells cultured in contact with 0.3 µM or 3 µM of urinastatin (Lot 67B8) as added to 10% fetal bovine serum-SHEM modified medium were definitely more numerous as compared with the urinastatin-free group and aprotinin-added group notwithstanding the fact that the culture supernatant had been removed prior to UV irradiation, thus leaving little reside of urinastatin at UV irradiation. The number of viable cells in the urinastatin 0.3 µM group was nearly equal to the number of control cells not irradiated, leading to the conclusion that urinastatin protected the cells almost completely against all injuries that might have been caused by ultraviolet radiation.

In the urinastatin 3 µM group, the number of viable cells was increased over the number of control cells not exposed to UV radiation. This result suggests that, in addition to the protective effect on cells against UV radiation, a promoting effect on the proliferation of cells, such as the effect demonstrated in Example 2, was expressed.

On the other hand, no cytoprotective effect was observed with aprotinin, but rather a tendency toward potentiating the lethal effect of UV light was observed. Therefore, the mechanism of protection by urinastatin is obviously different from the known protease inhibitory activity as observed with aprotinin.

Furthermore, when the above effect was evaluated by comparison with the hitherto-known free radical quenchers (suppressants), it is clear that urinastatin is very efficacious as can be seen from Table 8-2.

The 50% effective concentration of urinastatin in vitro for the quenching (suppression) of free radicals is reportedly about 1 µM [Advances in Medicine, 142, 895-896, 1987]. However, the concentration of urinastatin which proved effective in this example was 0.3 µM in the contact culture stage and, since the urinastatin-containing culture supernatant was discarded prior to UV exposure, the concentration of urinastatin at the UV irradiation stage where a large amount of free radicals is produced appears to have dropped to the order of less than a fraction. Even if it is supposed that 5% of urinastatin remained in the irradiation stage, its concentration should have been reduced to as low as 0.015 µM. It is, therefore, clear that urinastatin in such a very small amount is still capable of protecting the cells against ultraviolet radiation damage.

It is, therefore, clear that in the mechanism of cytoprotection by urinastatin against UV-induced damage, the role played by unknown factors other than the known free radical quenching (suppressive) action is of great significant.

It is also reported that urinastatin has the property to stabilize the lysosome membrane. Its protective effect on human corneal epithelial cells against ultraviolet radiation damage may also be suggested to be a protective action of urinastatin on the epithelial cell membrane as well.

To investigate the membrane breakdown in the respective cell groups used in Example 7, the lactate dehydrogenase (LDH) content of the culture supernatant in each cell group was determined before and after UV irradiation. As a result, LDH activity could not be detected in any of the control group, urinastatin-added group and aprotinin-added group.

In view of the possible LDH defection due to DNA damage, lysis of the cell membrane in each cell group was carried out with the surfactant Tween 80 and the LDH activity in the lyzate was assayed for confirmation. As a result, normal levels of LDH activity were found in all the cell groups. This finding indicates that the level of UV exposure used in Example 7 was not sufficient to induce breakdown of the cell membrane. The protective effect of urinastatin on epithelial cells against UV-induced damage was, thus, found to be not attributable to a cell membrane-protecting action which may be similar to the known lysosome membrane-stabilizing action of urinastatin.

The foregoing suggests that the principal mechanism of protection of human corneal epithelial cells by urinastatin against UV-induced damage is an unknown mechanism which does not correspond to any of the known 3 actions, namely (1) free radical quenching (suppressive) action, (2) protease-inhibitory action, and (3) lysosome membrane-stabilizing action.

Example 8

Efficacy of urinastatin in 193 nm UV-induced human corneal epithelial cell damage

Human corneal epithelial cell line HCEC was suspended in 10% fetal bovine serum-SHEM modified medium at a concentration of $1\times10^5$ cells/ml and 500 μl of this cell suspension was seeded into each well of a 24-well multiplate (Corning). The cells were cultured in a 5% $CO_2$ incubator at 37°C for 16 to 17 hours. After completion of culture, the supernatant was thoroughly discarded and 500 μl/well of 10% fetal bovine serum-SHEM modified medium containing the sample was added. The cells were further cultured in the presence of the sample under otherwise the same conditions as above for 2 hours. After completion of culture in contact with the sample, the culture supernatant was thoroughly removed so that substantially no residues of the sample were left behind in the multiwell plate.

The multiwell plate in this condition was irradiated with 193 nm UV light at a distance of 10 to 30 cm for 5 seconds. After the irradiation, 500 μl/well of 10% fetal bovine serum-SHEM modified medium containing the same sample as above was added and the cells were further cultured under the same conditions as above for 16 to 17 hours. Thereafter, in the same manner as in Example 7, the viable cells were counted and the protective effect of each of urinastatin (Lot 67B8) and aprotinin on human corneal epithelial cells against UV-induced damage was evaluated. The results are shown in Table 9. It is clear that urinastatin has a concentration-dependent cytoprotective effect. On the other hand, aprotinin showed no protective effect.

Table 9

| Efficacy of urinastatin in 193 nm UV-induced human corneal epithelial cell damage | |
| --- | --- |
| Sample (concentration μM) | Protective effect (%) |
| Urinastatin (0.3) | 134 |
| Urinastatin (3.0) | 145 |
| Aprotinin (0.3) | 94 |
| Aprotinin (3.0) | 83 |

Example 9

Efficacy of urinastatin in drug-induced human corneal epithelium cell damage

Human corneal epithelial cells (HCEC) were suspended in 10% fetal bovine serum-SHEM modified at a concentration of $1\times10^5$ cells/ml and 500 μl/well of this cell suspension was seeded on a 24-well multiplate (Corning). The cells were cultured in a 5% $CO_2$ incubator at 37°C for 24 hours. The culture supernatant was then discarded and after the cells in each well were washed with 500 µl of PBS(-) (Nissui Pharmaceutical) twice, the medium was replaced with 500 µl of the serum-free medium. The cells were further cultured in a 5% $CO_2$ incubator at 37°C for 12 hours. After completion of culture, the culture supernatant was discarded, the cells were washed with PBS(-) twice, and 500 µl of urinastatin (Lot 67B8)-containing serum-free medium was added. The cells were cultured in a 5% $CO_2$ incubator at 37 °C for 12 hours. After completion of culture, the supernatant was discarded, the wells were washed with PBS(-) twice, and 500 µl of the antiglaucoma drug Rescura (active ingredient: unoprostone isopropyl ester 0.12 mg/ml; Ueno Pharmaceutical) diluted with the serum-free medium beforehand to an unoprostone isopropyl ester concentration of 0.05 mg/ml was added. The plate was then allowed to sit at room temperature for 10 minutes. Thereafter, the culture supernatant was recovered and the LDH activity as an indicator of cytotoxicity was assayed. The results are shown in Table 10. It is clear that urinastatin showed a concentration-dependent anticytotoxic effect.

Table 10

| Efficacy of urinastatin in drug-induced human corneal epithelial cell damage | |
|---|---|
| Concentration of urinastatin (µg/ml) | LDH activity (absorbance at 570 nm, mean±SD |
| 0 | 0.336±0.06 |
| 1 | 0.304±0.06 |
| 10 | 0.065±0.05 |
| 100 | 0.018±0.07 |
| Control (Rescura not added) | 0.014±0.005 |

Example 10

Therapeutic efficacy of urinastatin in n-heptanol-induced rabbit corneal epithelial damage

Japanese white rabbits (Kitayama-Labes) weighing 2.5 to 3.0 kg were acclimatized for at least one week and submitted to the experiment after confirmation of the absence of any abnormality in the cornea etc. The experiment was performed in a controlled environment at room temperature: 23±3 °C and relative humidity: 50±20%. Ketamine hydrochloride was administered intramuscularly to each rabbit for general anesthesia and, then, 0.4% oxybuprocaine hydrochloride was instilled in the right eye for topical anesthesia. Then, an n-heptanol-saturated filter paper, 6 mm in diameter, was caused to contact the central surface of the cornea for 60 seconds to erode the epithelium to be detached, followed by inducing a lesion. Starting immediately after this epithelial excision, instillation of the test solution was carried out 6 times at 2-hour intervals for a total of 6 times (50 µl/dose). On the second day, starting 24 hours after excision, the instillation was carried out at 2-hour intervals for a total of 6 times. Saline was used as control. The area of the corneal epithelial lesion was measured by staining the lesion area with 0.5% fluorescein Na solution and photographing the stained area immediately after epithelial excision and, thereafter, at 12, 24, 30, 36 and 48 hours.
The effects on corneal epithelial wound healing are shown in Table 11. It is clear that up to 12 to 48 hours after excision, the instillation of urinastatin 1500 U/ml and 6000 U/ml promoted corneal wound healing as compared with the saline-instilled group.

Table 11

| Therapeutic efficacy of urinastatin in n-heptanol-induced rabbit corneal epithelial damage | | | |
|---|---|---|---|
| Time (hr.) after excision | Lesion area (%) | | |
| | Control (saline) | Urinastatin (1500 U/ml) | Urinastatin (6000 U/ml) |
| 0 | 100 | 100 | 100 |
| 12 | 89.3±4.6 | 80.6±5.4* | 79.3±7.3** |
| 24 | 57.6±6.8 | 43.7±7.9*** | 46.7±4.9** |
| 30 | 44.5±10.5 | 25.6±8.5*** | 29.6±6.1** |
| 36 | 36.8±11.3 | 12.3±7.4*** | 18.2±7.1*** |
| 48 | 16.2±13.3 | 1.5±1.4* | 3.4±4.3* |
| The corneal lesion area immediately after excision of the corneal epithelium (0 hr) is taken as 100%. Each value denotes mean±SD (n=7). | | | |

*p<0.05,
**p<0.01,
***p<0.001 vs. control

Example 11

Efficacy of urinastatin in UV-induced human conjunctival epithelial cell damage

Human conjunctival cells ATCC CCL20.2 were suspended in 10% fetal bovine serum-DMEM/F12 medium at a concentration of $1\times10^5$ cells/ml and 500 μl/well of this cell suspension was seeded on a 24-well multiwell plate (Sumitomo Bakelite) and cultured in a 5% $CO_2$ incubator at 37 °C for 16 to 17 hours. The culture supernatant was discarded thoroughly and 500 μl/well of 10% fetal bovine serum-DMEM/F12 medium containing a test sample (urinastatin Lot 67B8; final concentration 100 μg/ml) was added. The cells were further cultured under the same conditions as above. After completion of culture in contact with the sample, the culture supernatant was thoroughly removed. The multiwell plate in this condition was irradiated with UV-B (312 nm, 100 mJ/cm$^2$). After UV-B irradiation, 500 μl/well of 10% fetal bovine serum-DMEM/F12 medium was added and the cells were cultured under the same conditions as above for 16 to 17 hours. Then, 1/10 volume of WST-8 solution (Cell Counting Kit-8, Dojin Chemical) was added to the medium and the reaction was carried out at 37 °C for 4 hours. Then, the absorbance at 450 nm of the water-soluble formazan produced by intracellular dehydrogenase activity was measured to estimate the viable cell count. Then, the cytoprotective effect was evaluated by the same computation as in Example 7. The results are shown in Table 12. It is clear that urinastatin has a protective effect against UV-induced damage.

Table 12

| Efficacy of urinastatin in UV-induced human conjunctival epithelial cell damage | | |
|---|---|---|
| | Cell proliferation (A450 nm, mean±SD) | Protective effect (%) |
| Urinastatin (0.3 μM) | 0.556±0.015 | 173 |
| Urinastatin (3 μM) | 0.765±0.009 | 238 |

Example 12

Efficacy of urinastatin in drug-induced human conjunctival epithelial cell damage

A 48-well plate was seeded with 300 μl/well of a suspension of human conjunctival epithelial cells ATCC CCL20.2 in 10% fetal bovine serum-DMEM/F12 medium ($1\times10^5$/ml) and the cells were cultured in a 5% $CO_2$ incubator at 37 °C.

After 48 hours, confirming that the cells had become fully confluent, the supernatant was discarded and the cells were washed with PBS (-) twice and suspended in serum-free DMEM/F12 medium. The cells were incubated under 5% $CO_2$ at 37 °C for 12 hours, after which the supernatant was discarded again. The cells, were washed with PBS (-) twice and 300 μl of urinastatin (Lot 67B8) diluted with PBS(-) beforehand to a varying concentration was added. The cells were incubated under 5% $CO_2$ at 37 °C for 12 hours and the supernatant was discarded again. The cells were then washed with PBS(-) twice and 300 μl of the antiglaucoma drug Timoptol (active ingredient timolol maleate 50 mg/ml; Banyu Pharmaceutical) diluted with PBS (-) beforehand to a timolol maleate concentration of 1.25 mg/ml was added. After 20 minutes, the plate was washed again with PBS (-) twice and 300 μl of DMEM/F12 was added. After 12 hours, WST-8 was added and reacted at 37 °C for 2 hours. The absorbance at 450 nm was then measured to estimate the number of viable cells. The results are shown in Table 13. It is clear that urinastatin shows a concentration-dependent cytoprotective effect.

Table 13

| Efficacy of urinastatin in drug-induced human conjunctival epithelial cell damage | |
| --- | --- |
| Concentration of urinastatin (μg/ml) | Cell proliferation (A450 nm, mean±SD) |
| Control (no drug added) | 0.738±0.025 |
| 0 | 0.277±0.090 |
| 1 | 0.577±0.120 |
| 10 | 0.686±0.065 |
| 100 | 0.747±0.096 |
| n=6 | |

Example 13

Effect of urinastatin on the mucin 1 production of human conjunctival epithelial cells

Human conjunctival epithelial cells (ATCC CCL20.2) were suspended in 10% fetal bovine serum-DMEM/F12 medium (Gibco) and seeded on a 96-well plate, 5000 cells/well. The cells were cultured in a 5% $CO_2$ incubator at 37 °C overnight to cause adhesion of the cells on the plate. The medium was then replaced with the same medium containing the sample and the cells were cultured for a further 2 days. Thereafter, the output of mucin 1 was determined by the following enzyme immunoassay. Thus, after the cells were fixed with 70% ethanol, 250 μl/well of 1% bovine serum albumin-PBS was added and the plate was allowed to sit at room temperature for 2 hours. Then, anti-mucin 1 mouse antibody (Pharmingen) diluted with PBS was added and reacted at room temperature for 2 hours. The plate was washed with 3 PBS 3 times. Peroxidase-labeled anti-mouse IgG antibody (Santa Cruz Biotechnology) diluted with PBS beforehand was then added and after 2 hours of reaction at room temperature, the plate was washed with PBS 4 times. Then, 200 μl of a peroxidase substrate (0.1 M phosphate buffer containing 0.012% hydrogen peroxide and 1.6 mg/ml orthophenylenediamine, pH 6.2) was added and the reaction was carried out at room temperature for 20 minutes, at the end of which time the reaction was stopped by adding 50 μl of 4.5 N-sulfuric acid. The absorbance (490 nm - 660 nm) was measured with a microplate reader to estimate the output of mucin 1. The results are shown in Table 14. The mean absorbance value of the control culture using the urinastatin-free medium was taken as 100%. It is clear that urinastatin shows a concentration-dependent promoting effect on mucin 1 production.

Table 14

| Effect of urinastatin on the mucin 1 production of human conjunctival epithelial cells | |
|---|---|
| Sample (addition amount μg/ml) | Production of mucin 1 (%±SE) |
| Not added (0) | 100 |
| Urinastatin (50) | 111±6.0 |
| Urinastatin (200) | 123±6.8* |
| n = 5, | |

*$p < 0.05$

Example 14

Effect of urinastatin on keratoconjunctival epithelial impairment in a rat forced-open air-draft dry eye model

Male Sprague-Dawley rats aged 6 weeks were used. It was confirmed in advance that there was no abnormality in the anterior eye segment and the eyelids of animals. In the left eye of 10 rats, a solution of urinastatin (Lot 67B8 diluted to 500 μg/ml with saline) was instilled, 5 μl per dose, 6 times daily for 1 week. In the control right eye, saline was similarly instilled. Two hours after the last instillation, the keratoconjunctiva was dehydrated by the following method to induce a keratoconjunctival epithelial damage and the effect of urinastatin on the lesion was evaluated.

Under anesthesia with pentobarbital 40 mg/kg (i.p.), the eyelids of ten rats treated as above were sufficiently retracted to open, and using a dryer held at a distance of 6 cm from the front of the eye, the entire anterior segment of the eye was exposed to a draft of air for 15 minutes to vaporize the tears off and dehydrate the keratoconjunctiva, whereby a keratoconjunctival epithelial lesion was induced. After induction of epithelial damage, 5 μl of 1% Rose Bengal solution was instilled, followed by thorough washing with saline, whereby the mucin-defective part of the keratoconjunctiva was stained. The animal was then sacrificed by administering an overdose of pentobarbital i.v. and the eyeglobe inclusive of the bulbar conjunctiva was carefully enucleated and immediately submitted to observation of the lesion not covered with the Rose Bengal-stained mucin. The positive Rose Bengal stain was quantitated by scoring the degree of staining for each of the nasal side and aural side of the bulbar conjunctiva and the cornea on a scale of 0 to 3 points, or a total of 9 points, according to the criteria for Diagnosis of A Dry Eye (Dry Eye Study Group, 1995). The higher the score is, the higher is the severity of keratoconjunctival epithelial damage. This scoring was carried out by a single examiner and the evaluation was made by masking the distinction between the right eyes and the left eyes.

The results are shown in Table 15. In the 500 μg/ml urinastatin instillation group, as compared with the saline instillation group, the keratoconjunctival damage by dehydration was significantly suppressed ($P<0.05$). It is, thus, clear that urinastatin inhibits the mucin covering defect of the keratoconjunctiva in a dry eye.

Table 15

| Efficacy of urinastatin in keratoconjunctival epithelial impairment associated with dry eye Evaluation scores in bulbar keratoconjunctiva (rats) | | |
|---|---|---|
| Score | Urinastatin | Control |
| 9 | 0 | 0 |
| 8 | 0 | 1 |
| 7 | 0 | 0 |
| 6 | 0 | 2 |
| 5 | 1 | 0 |
| 4 | 0 | 2 |
| 3 | 2 | 1 |

Table 15 (continued)

| Efficacy of urinastatin in keratoconjunctival epithelial impairment associated with dry eye Evaluation scores in bulbar keratoconjunctiva (rats) | | |
|---|---|---|
| Score | Urinastatin | Control |
| 2 | 1 | 3 |
| 1 | 5 | 1 |
| 0 | 1 | 0 |
| Mean score | 1.8±1.5 | 3.8±2.3 |

Production Example 1

| Urinastatin (specific activity 4000 to 2000 units/mg protein) | 50 mg protein |
|---|---|
| Chlorobutanol | 10 mg |
| Sodium chloride | 240 mg |
| Sodium dihydrogenphosphate | 240 mg |
| Sodium monohydrogenphosphate | 200 mg |

The above ingredients were dissolved in 100 ml of sterile distilled water and sterilized by a membrane filter and the filtrate was distributed into sterilized ophthalmic dispensing bottles, 10 ml per bottle, to provide an ophthalmic solution.

INDUSTRIAL APPLICABILITY

The therapeutic composition for corneal epithelial disorders according to the present invention is of use as a prophylactic and therapeutic drug for corneal epithelial disorders in mammals including man, namely herpetic keratitis, bacterial corneal ulcer, neuroparalytic keratitis, diabetic keratopathy, and corneal epithelial disorders arising from physical or chemical injuries.

The therapeutic composition for UV-induced keratoconjunctival epithelial disorders according to the present invention is of use as a prophylactic and therapeutic drug for ultraviolet radiation-induced keratoconjunctival epithelial disorders.

The therapeutic composition for keratectomy postoperative corneal epithelial complications according to the present invention is of use as a prophylactic and therapeutic drug for postoperative corneal epithelial complications in keratectomized cases.

Furthermore, the therapeutic composition for drug-induced keratoconjunctival epithelial disorders according to the present invention is of use as a therapeutic drug for drug-induced keratoconjunctival epithelial disorders which is capable of getting rid of side effects of ophthalmic drugs having a keratoconjunctival epithelium-impairing potential and enabling said ophthalmic drugs to be properly indicated.

Finally, the therapeutic composition for dry eyess according to the present invention is of use as a prophylactic and/or therapeutic drug for dry eyes.

**Claims**

1.  A therapeutic composition for corneal epithelial disorders which is indicated for the prophylaxis and/or therapy of corneal epithelial disorders,
    comprising urinastatin as an active ingredient.

2.  A therapeutic composition for UV-induced keratoconjunctival epithelial disorders which is indicated for the prophylaxis and/or therapy of UV-induced disorders of the keratoconjunctival epithelia,
comprising urinastatin as an active ingredient.

3. A therapeutic composition for postoperative corneal epithelial complications which is indicated for the prophylaxis and/or therapy of postoperative corneal epithelial complications in keratectomized cases,
comprising urinastatin as an active ingredient.

4. A therapeutic composition for drug-induced keratoconjunctival epithelial disorders which is indicated for the therapy of keratoconjunctival epithelial disorders arising from administration of an ophthalmic drug,
comprising urinastatin as an active ingredient.

5. A pharmaceutical composition for dry eyes which is indicated for the prophylaxis and/or therapy of dry eyes,
comprising urinastatin as an active ingredient.

Fig. 1

Control

Urinastatin (0.5 μg/ml)

Urinastatin (5 μg/ml)

# INTERNATIONAL SEARCH REPORT

| International application No. |
| --- |
| PCT/JP99/00853 |

| A. CLASSIFICATION OF SUBJECT MATTER |
| --- |
| Int.Cl⁶ A61K38/57 |
| According to International Patent Classification (IPC) or to both national classification and IPC |

| B. FIELDS SEARCHED |
| --- |
| Minimum documentation searched (classification system followed by classification symbols)<br>Int.Cl⁶ A61K38/57 |
| Documentation searched other than minimum documentation to the extent that such documents are included in the fields searched |
| Electronic data base consulted during the international search (name of data base and, where practicable, search terms used)<br>CAPLUS (STN), MEDLINE (STN) |

C. DOCUMENTS CONSIDERED TO BE RELEVANT

| Category* | Citation of document, with indication, where appropriate, of the relevant passages | Relevant to claim No. |
| --- | --- | --- |
| A | JP, 63-267730, A (Tadamasa Kosugi),<br>4 November, 1988 (04. 11. 88),<br>Particularly refer to Text Example 2, "Allergy sei ketsumakuen ni taisuru sayou" (Family: none) | 1-5 |
| A | EP, 223254, A2 (LABOSYSTEMS OY),<br>27 May, 1987 (27. 05. 87),<br>Cited in the present application, refer to the full text & US, 4849406, A & JP, 62-187413, A | 1-5 |

| ☐ Further documents are listed in the continuation of Box C. | ☐ See patent family annex. |
| --- | --- |

| * Special categories of cited documents: | "T" later document published after the international filing date or priority date and not in conflict with the application but cited to understand the principle or theory underlying the invention |
| --- | --- |
| "A" document defining the general state of the art which is not considered to be of particular relevance | |
| "E" earlier document but published on or after the international filing date | "X" document of particular relevance; the claimed invention cannot be considered novel or cannot be considered to involve an inventive step when the document is taken alone |
| "L" document which may throw doubts on priority claim(s) or which is cited to establish the publication date of another citation or other special reason (as specified) | |
| "O" document referring to an oral disclosure, use, exhibition or other means | "Y" document of particular relevance; the claimed invention cannot be considered to involve an inventive step when the document is combined with one or more other such documents, such combination being obvious to a person skilled in the art |
| "P" document published prior to the international filing date but later than the priority date claimed | "&" document member of the same patent family |

| Date of the actual completion of the international search<br>7 June, 1999 (07. 06. 99) | Date of mailing of the international search report<br>15 June, 1999 (15. 06. 99) |
| --- | --- |
| Name and mailing address of the ISA/<br>Japanese Patent Office | Authorized officer |
| Facsimile No. | Telephone No. |

Form PCT/ISA/210 (second sheet) (July 1992)